# FASCICULE DE BREVET EUROPEEN

(11) **EP 1 991 437 B1**
(45) Date de publication et mention de la délivrance du brevet: **19.05.2010**
(21) Numéro de dépôt: 07731694.1
(22) Date de dépôt: 05.03.2007
(51) Int. Cl.: B60K 15/04, G01N 33/22

(54) **CIRCUIT CARBURANT D'UN VEHICULE**
KRAFTSTOFFSYSTEM FÜR EIN FAHRZEUG
FUEL SYSTEM OF A VEHICLE

(30) Priorité: 06.03.2006 FR 0650779
(43) Date de publication de la demande: 19.11.2008
(73) Titulaire: PEUGEOT CITROËN AUTOMOBILES S.A., 78140 Vélizy Villacoublay (FR)
(72) Inventeur: HADDAD, Michaël, F-91190 Gif-sur-Yvette (FR); SADIK-ROZSNYAI, Orsolya, F-92400 Courbevoie (FR); DE BATZ DE TRENQUELLEON, Marc, F-92330 Sceaux (FR)
(74) Mandataire: Ménès, Catherine
(86) Numéro de dépôt international: PCT/FR2007/050877
(87) Numéro de publication internationale: WO 2007/101960

(56) Documents cités:
- EP-A2- 1 134 574
- WO-A-00/33039
- WO-A-2007/004897
- DE-A1- 10 322 300
- DE-A1- 10 358 619
- DE-A1-102005 001 716
- FR-A1- 2 632 408
- US-A1- 2003 043 378
- ANONYMOUS: "Spectrophotometry" WIKIPEDIA, THE FREE ENCYCLOPEDIA, [Online] XP002419696 Extrait de l'Internet: URL:http://en.wikipedia.org/wiki/Spectroph otometry> [extrait le 2007-02-13]

## Description

La présente invention revendique la priorité de la demande française 0650779 déposée le 06/03/2006.

La présente invention concerne l'identification des caractéristiques de bases d'un carburant automobile, en particulier la discrimination au niveau du véhicule entre les carburants de type «essence » et les carburants du type « diesel » en fonction du type de moteur équipant le véhicule.

Le marché des véhicules automobiles pour particuliers et celui des petits véhicules utilitaires est essentiellement partagé entre des véhicules équipés d'un moteur à combustion interne commandée, communément appelés moteurs essence, et des véhicules équipés d'un moteur à combustion interne spontanée, du type Diesel qui nécessitent un carburant du type gazole. Ces dernières années, il a été constaté une forte recrudescence des confusions entre carburants dans les stations service. En effet, de nombreux ménages disposent de plusieurs véhicules qui peuvent être équipés de motorisations différentes. De plus, les flottes (véhicules de locations, véhicules de service, etc.) se sont beaucoup développées de sorte que les automobilistes utilisent tour à tour des véhicules nécessitant du gazole ou de l'essence ce qui génère des risques d'erreurs.

Les pompes distributrices sont en principe équipées d'un détrompeur mécanique sensé interdire le remplissage d'un réservoir essence par du gazole, la section du pistolet d'une pompe gazole étant plus large que l'entrée du réservoir dans un véhicule à moteur essence. Néanmoins, des utilisateurs distraits prouvent chaque jour que ce détrompeur n'est pas infaillible.

A l'évidence, ce système mécanique n'est d'aucune efficacité pour prévenir le remplissage accidentel d'un réservoir de gazole avec de l'essence. Dans les pays où la pénétration du marché des véhicules à moteurs Diesel est relativement récente, ces erreurs se répètent plus de cent mille fois par an. Or, le gazole est un carburant sélectionné en fonction de ses propriétés de lubrification, propriétés indispensables pour limiter l'usure des pièces mobiles d'un moteur Diesel. De plus, les systèmes d'injection modernes du type injection directe haute pression sont également très sensibles à la nature du carburant. L'utilisation d'essence à la place de gazole peut donc provoquer au pire une casse moteur et dans le meilleur des cas, une destruction de pièces telles la pompe à carburant, la pompe à injection haute pression ou des injecteurs.

Par ailleurs, si la panne ne survient pas immédiatement, ce qui est notamment le cas si le réservoir du véhicule n'était pas quasiment vide au moment de son remplissage, la confusion de carburant met en danger la sécurité des occupants du véhicule, et des autres véhicules circulant, car elle peut entraîner une coupure moteur totalement inattendue et pratiquement instantanée.

Si l'automobiliste constate son erreur au niveau même de la pompe, il en sera quitte avec une purge du circuit d'alimentation et un nouveau plein avec le carburant adapté, soit essentiellement une simple perte de temps sans vraie conséquence financière ou autre. De nombreuses études ont de ce fait été poursuivies visant à empêcher l'introduction d'un carburant inadapté ou à alerter l'automobiliste si tel est le cas.

Il a ainsi été proposé par exemple dans le brevet FR2741014 des dispositifs détrompeurs mécaniques pour empêcher l'introduction dans l'orifice du réservoir du véhicule un pistolet ayant un diamètre de plus petit que la taille normale d'un pistolet gazole. Plus récemment, il a été proposé dans la demande de brevet WO2004101305A1 des moyens pour mesurer la taille du pistolet associés à des moyens bloquant l'introduction du pistolet si celui-ci n'est pas à la bonne taille.

D'autres dispositifs, connus par exemple du brevet US5309957, sont associés au pistolet, et comportent des capteurs permettant de vérifier que le carburant avec lequel l'automobiliste s'apprête à faire le plein correspond bien au carburant déjà dans le véhicule, et à stopper la pompe distributrice de carburant si tel n'est pas le cas. A l'évidence, ce type de dispositif n'est efficace que si l'automobiliste s'approvisionne auprès de stations services équipées.

Enfin, il a également été proposé d'équiper le véhicule automobile de capteurs propres à déterminer la nature du carburant. Ainsi, il est connu de la demande de brevet EP1134574A2 d'équiper la conduite entre la trappe carburant et le réservoir d'un véhicule de moyens pour prélever un volume de carburant et le peser, associer à des moyens pour empêcher le démarrage du véhicule si le carburant n'est pas idoine.

Ce type de dispositif suppose une modification du circuit carburant pour y associer des moyens de prélèvement du carburant.

Il subsiste donc un besoin d'un moyen qui soit à la fois peu coûteux, autonome, notamment non lié au réseau de distribution en carburant, indépendant du moyen utilisé pour remplir le réservoir, donc pouvant être utilisé quel que soit le type de pompe à carburant ou même en l'absence de pompe (l'utilisateur doit pouvoir remplir son réservoir à partir d'un jerrican en cas de besoin) et à la fois fiable et rapide pour alerter l'automobiliste le plus rapidement possible.

La possibilité d'une analyse colorimétrique est connue de DE 10358619, qui divulge toutes les caractéristiques du préambule de la revendication 1 et dans laquelle ce problème est résolu par l'implantation dans le circuit carburant d'un moyen d'analyse colorimétrique du carburant.

L'essence et le gazole différent beaucoup de part leurs propriétés physiques et chimiques. L'essence est un mélange de molécules d'hydrocarbures à chaîne carbonée « courte » (entre 1 et 13 atomes de carbone) alors que le gazole est un carburant « lourd »composé d'hydrocarbures à chaîne beaucoup plus longue et complexe. Cette différence de composition se retrouve dans les caractéristiques physico-chimiques (capacité calorifique, pression de vapeur saturante, viscosité, densité, ....) de ces carburants.

La couleur est également au nombre de ces propriétés physico-chimiques qui permettent de distinguer une essence et un gazole. Les gazoles ont une teinte jaunâtre, allant selon les zones géographiques de commercialisation du jaune clair au marron. Les essences sont elles essentiellement transparentes, pratiquement comme de l'eau avec éventuellement un peu d'effet de diffusion lors donnant un aspect translucide.

Cette différence de couleur s'explique par l'absorption des hydrocarbures qui composent ces carburants. Dans l'essence, les chaînes carbonées sont courtes et l'absorption liée aux transitions électroniques centrée dans le proche ultraviolet vers 300 nm alors que dans le cas des gazoles cette dernière est centrée autour de 360 nm, dans le violet, et s'étend jusque dans la composante bleue du spectre visible. C'est l'absorption de la composante bleue du spectre par les longues chaînes carbonées qui composent un gazoles qui expliquent sa couleur jaunâtre (les composantes rouges et vertes du spectre ne sont pas absorbées).

Il doit être noté que même si certains carburants diesel présentent une très faible coloration (cas par exemple des gazoles commercialisés dans certaines régions du globe comme le Japon ou la Martinique), ils absorbent néanmoins dans le bleu (ou plus exactement comme indiqué précédemment dans violet ou l'ultraviolet) - contrairement à des essences même légèrement teintées. Dans ces cas, il s'agit donc plus d'une analyse spectroscopique que d'une analyse colorimétrique proprement dite, la différenciation essence/gazole étant réalisée sur la base des différences d'absorption dans les longueurs d'onde comprises entre environ 350 nm et environ 450 nm.

A côté de cette plage d'absorption dans le bleu, la mesure peut également être effectuée dans le proche infrarouge, domaine dans lequel les propriétés d'absorption du gazole et de l'essence diffèrent d'environ un facteur 2, c'est-à-dire d'un facteur plus petit que celui constaté dans le bleu mais néanmoins suffisant pour discriminer l'essence ou le gazole.

L'absorption de la lumière peut être observée en transmission, par exemple en plaçant la source lumineuse et le capteur se faisant face selon un diamètre du conduit raccordant la trappe à carburant du véhicule au réservoir. Elle peut-être également en réflexion, auquel cas source et capteur peuvent être disposer à proximité immédiate l'un de l'autre.

La présente invention se propose donc d'exploiter ce phénomène en proposant d'éclairer le carburant avec une lumière bleue et en observant l'absorption de cette lumière par le carburant.

Cette méthode peut être mise en oeuvre avec des moyens très simples tant au niveau des sources lumineuses que des détecteurs. Ainsi un simple phototransistor associé à une diode électroluminescente (LED) bleue est suffisant.

Dans une variante plus particulièrement préférée de l'invention, la mesure effectuée est essentiellement par diffusion et absorption, méthode préférée car compatible avec un fluide ayant un fort degré de turbulence, comme il est souvent le cas avec un carburant juste au sortir d'une pompe.

Au système d'identification du carburant vient de préférence s'ajouter des moyens pour avertir l'automobiliste de l'erreur du carburant, par exemple par le déclenchement d'une alarme sonore l'invitant à stopper immédiatement le remplissage du réservoir et/ou des moyens pour bloquer le démarrage du moteur afin d'empêcher la destruction des éléments sensibles, notamment si l'alarme n'a pas été prise en compte dans un bref délai.

L'invention sera bien comprise à la lecture de la description qui va suivre, donnée uniquement à titre d'exemple et faite en se referant aux dessins annexes, parmi lesquels :

la figure 1 est un schéma de du circuit carburant d'un véhicule automobile, de la trappe carburant au réservoir ;

la figure 2 est un schéma de montage connu en transmission ;

la figure 3 est un schéma de montage connu du DE 102005001716 en réflexion ;

la figure 4 est un schéma illustrant l'invention, avec une mesure selon le mode diffusion/absorption.

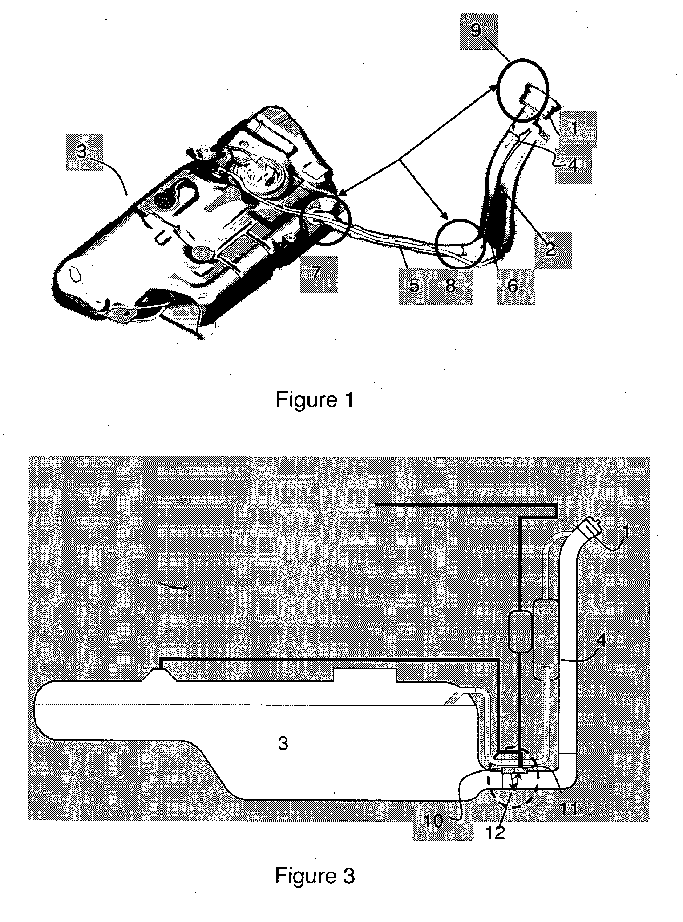

Comme il est illustré à la figure 1, le circuit d'arrivée du carburant comporte essentiellement une embouchure 1, terminée par un bouchon et/ou une trappe, un conduit 2 et un réservoir 3. Sur les véhicules modernes, le réservoir est typiquement placé sous le plancher du véhicule, de sorte que le conduit 2 comporte une première partie 4 essentiellement verticale et une seconde partie 5 essentiellement horizontale, ces deux parties étant jointes par un coude 6.

Sur cette figure 1, on a entouré trois points privilégiés d'implantation d'un capteur selon l'invention, encerclés sur cette figure et situés respectivement au niveau du point d'entrée 7 dans le réservoir, au niveau du coude 8 et au niveau de la trappe 9. A noter que ces points sont des zones de soudure ce qui permet d'insérer simplement l'émetteur et le détecteur au moment de la fabrication du conduit. Plus on se rapproche du réservoir, plus le flux de carburant est homogénéisé ce qui est favorable à la mesure. Pour autant, une mesure relativement en aval du circuit carburant s'accomplit alors qu'une quantité non négligeable de carburant a été admise dans le circuit, de sorte que pour maintenir des performances motrices idéales, une vidange complète du circuit carburant doit être recommandée à l'automobiliste. Une mesure en amont, juste au niveau de la trappe, permet d'alerter l'automobiliste au plus vite.

Selon l'invention, l'identification du carburant s'effectue essentiellement par calorimétrie, ou plus précisément, est basée sur la considération selon laquelle le gazole absorbe une lumière bleue. Pour être tout à fait exact, l'optimum d'absorption est autour de 360 nm, c'est-à-dire dans le violet. Pour autant, l'absorption de la lumière est quasiment totale pour toute lumière dont la longueur d'onde est inférieure est comprise entre environ 300 nm et environ 500 nm, ce qui permet d'utiliser toute source lumineuse émettant dans le « bleu », notamment une diode électroluminescente comme on en trouve pour de multiples applications commerciales et qui émettent selon un spectre plus ou moins large. Bien évidemment, une source monochromatique, notamment une source laser, peut être également utilisée.

La détection du rayon lumineux peut être réalisée par tout moyen propre à détecter une lumière non absorbée. Un phototransistor sera de préférence utilisé en raison de son faible coût mais d'autres moyens, en particulier une photodiode ou un joulemètre peuvent être également utilisés.

Dans tous les cas, la source lumineuse et le détecteur pourront de préférence placés dans un point du conduit nécessitant de toute façon une soudure.

Comme indiqué précédemment, la lumière bleue est essentiellement absorbée dans le gazole alors qu'elle est transmise essentiellement non absorbée dans l'essence. Autrement dit, la mesure peut être considérée comme une mesure du type « tout ou rien », ne nécessitant qu'un circuit électronique élémentaire pour transmettre par exemple l'information « essence détectée » lorsque le véhicule est équipé d'un moteur Diesel, par exemple sur la base d'un étalonnage.

Cette information peut être traitée de différentes façons. Par exemple, elle peut être associée à un avertisseur sonore et/ou visuel (mise en route des feux clignotants d'avertisseur d'urgence) et à une interdiction de démarrage. Des stratégies mixtes peuvent avantageusement être utilisées. Par exemple, si l'utilisateur réagit immédiatement au signal d'alerte et stoppe le remplissage de son réservoir, puis le poursuit cette fois avec un carburant adapté, il peut être estimé que la quantité de «mauvais » carburant est minime et non nuisible.

Si par contre, l'utilisateur continue l'opération de remplissage ou s'arrête sans pour autant continuer ultérieurement le remplissage avec un « bon » carburant (selon les informations communiquées par la jauge), alors le redémarrage du véhicule peut être interdit.

En tout état de cause, l'interface avec le calculateur pour un traitement des données et l'application de la stratégie d'avertissement de l'usager est facilitée du fait de la présence d'un faisceau de communication pour l'information jauge carburant à proximité.

Le circuit électrique d'alimentation de la source lumineuse est de préférence fermé dès que la trappe carburant est ouverte. De préférence encore, la lumière est émise sous forme d'impulsions plutôt que de façon continue, ce qui permet de différencier une absorption quasi nulle (le conduit ne contient que de l'air), d'une absorption très légère (le conduit est rempli avec une essence et non un gazole).

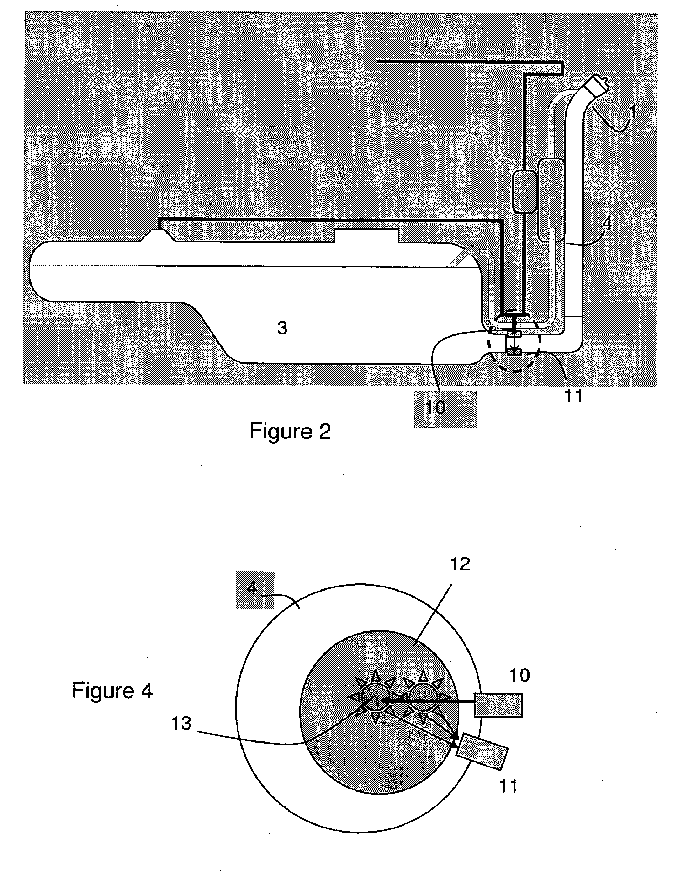

Comme déjà connu la mesure peut être effectuée en transmission comme illustré à la figure 2. L'émetteur 10 et le détecteur 11 sont en vis-à-vis par exemple diamétralement opposés ou éventuellement très légèrement décalés et sont séparés par une épaisseur de carburant correspondant au diamètre du conduit carburant. Dans ce montage, La lumière traverse une seule fois le carburant. Cet unique passage n'est pas très favorable du point de vue de la sensibilité du dispositif mais permet d'espacer émetteur et détecteur d'une épaisseur de liquide (carburant) relativement importante, par exemple de plus de 100 mm.

Comme également déjà connu et illustrée à l'aide de la figure 3, la mesure peut être effectuée en réflexion. Il est nécessaire de disposer d'une surface réfléchissante 12 en regard de la source lumineuse, par exemple constituée d'un miroir métallique ou diélectrique ou même plus simplement d'une simple surface blanche. Le signal issu de l'émetteur 10 est réfléchi par cette surface réfléchissante et revient vers le détecteur 11 positionné du même côté que l'émetteur (le miroir pouvant éventuellement être placé de manière telle que le faisceau lumineux soit légèrement dévié, notamment dans l'hypothèse d'une source lumineuse émettant un pinceau lumineux très fin et d'un détecteur non superposé à la source. A noter qu'en cas d'utilisation d'une diode électroluminescente, la dispersion du faisceau lumineux est suffisante pour autoriser un léger décalage entre la source émettrice et le détecteur.

Comme le signal effectue un trajet aller et retour, la sensibilité est accrue mais la distance entre l'émetteur et la paroi réfléchissante ne doit pas être trop importante.

Selon l'invention, applicable en un point du conduit où le carburant conduit une quantité d'air relativement importante, autrement dit à proximité de la trappe à essence, juste sous le pistolet de la pompe à carburant, on utilise le principe combiné de la diffusion et de l'absorption. En effet l'air continu dans le carburant va provoquer une diffusion importante du signal.

Comme illustré à l'aide de la figure 4, ce phénomène peut être exploité en plaçant le détecteur du même côté que l'émetteur, à une distance n' excédant pas par exemple 90° d'arc de cercle, et de préférence comprise entre environ 10° et environ 60°. Dans ce cas, il n'est pas nécessaire de prévoir de surface réfléchissante.

Tant que le conduit 4 est rempli d'air, le signal lumineux n'atteint pas le détecteur. A partir du moment où un jet 12 de carburant est introduit, mélange d'air et de carburant comme au sortir d'un pistolet de carburant, l'air 13 contenu dans le carburant provoque une diffusion importante du signal lumineux dont une partie arrive alors au détecteur. Si le carburant est du type essence cette partie du signal est diffusée sans être absorbée et donc un signal de niveau élevé est détecté. Si le carburant est du type gazole, l'absorption du signal est très importante et donc seul un faible signal est détecté.

L'invention convient tout particulièrement à une détection très précoce de la nature du carburant car elle est d'autant plus efficace que le carburant n'a pas eu le temps de décanter, autrement dit que la mesure est effectuée juste au niveau de l'extrémité du pistolet de la pompe à carburant, lorsque celui-ci est introduit au maximum dans le conduit.

## Revendications

1. Circuit carburant d'un véhicule automobile comportant des moyens d'analyse colorimétrique du carburant pour la discrimination entre une essence et un gazole, **caractérisé en ce que** la mesure est effectuée en un point du circuit où le carburant est trouble et **en ce que** le circuit comporte une source lumineuse émettant dans le bleu et un détecteur, placé à une distance angulaire de la source lumineuse comprise entre 10° et 90°, de sorte que le signal diffusé par l'air emprisonné dans le carburant est transmis, en étant plus ou moins absorbé selon la nature du carburant, au détecteur.

2. Circuit carburant selon la revendication 1, **caractérisé en ce que** le détecteur est choisi parmi les moyens suivants : photodiode, phototransistor et joulemètre.

3. Circuit carburant selon l'une quelconque des revendications 1 ou la revendication 2, **caractérisé en ce que** la source lumineuse est une diode électroluminescente.

4. Circuit carburant selon l'une quelconque des revendications précédentes, associé à des moyens d'avertissement sonores ou visuels si le carburant utilisé pour remplir le réservoir n'est pas adapté au moteur du véhicule.

5. Circuit carburant selon l'une quelconque des revendications précédentes associé à des moyens pour bloquer le redémarrage du véhicule si le carburant utilisé pour remplir le réservoir n'est pas adapté au moteur du véhicule.

## Claims

1. Fuel circuit of an automobile vehicle comprising means for the colorimetric analysis of the fuel for discrimination between a petrol fuel and a diesel fuel, **characterized in that** the measurement is carried out at a point of the circuit where the fuel is turbid and **in that** the circuit comprises a light source emitting in the blue range and a detector, placed at an angular distance from the light source comprised between 10° and 90°, such that the signal diffused by the air trapped in the fuel is transmitted, by being more or less absorbed according to the nature of the fuel, to the detector.

2. Fuel circuit according to Claim 1, **characterized in that** the detector is selected from the following means: photodiode, phototransistor and joulemeter.

3. Fuel circuit according to any of Claims 1 or Claim 2, **characterized in that** the light source is a light-emitting diode.

4. Fuel circuit according to any of the preceding claims, associated with audio or visual warning means if the fuel which is used for filling the tank is not suited to the engine of the vehicle.

5. Fuel circuit according to any of the preceding claims associated with means to block the re-starting of the vehicle if the fuel used to fill the tank is not suited to the engine of the vehicle.

## Patentansprüche

1. Kraftstoffkreislauf eines Kraftfahrzeugs, der Farbanalysemittel des Kraftstoffs zum Unterscheiden zwischen Benzin und Diesel aufweist, **dadurch gekennzeichnet, dass** die Messung an einer Stelle des Kreislaufs erfolgt, an der der Kraftstoff trüb ist, und dass der Kreislauf eine Lichtquelle aufweist, die im blauen Bereich sendet, sowie einen Detektor, der in einer Winkelentfernung von der Lichtquelle angeordnet ist, die zwischen 10° und 90° liegt, so dass das von der in dem Kraftstoff gefangenen Luft verteilte Signal übertragen wird, indem es je nach Beschaffenheit des Kraftstoffs am Detektor mehr oder minder absorbiert wird.

2. Kraftstoffkreislauf nach Anspruch 1, **dadurch gekennzeichnet, dass** der Detektor aus den folgenden Mitteln ausgewählt wird: Fotodiode, Fototransistor oder Joulemeter.

3. Kraftstoffkreislauf nach einem der Ansprüche 1 oder 2, **dadurch gekennzeichnet, dass** die Lichtquelle eine lichtemittierende Diode ist.

4. Kraftstoffkreislauf nach einem der vorhergehenden Ansprüche, verbunden mit Mitteln zum akustischen oder visuellen Warnen, wenn der zum Füllen des Tanks verwendete Kraftstoff für den Motor des Fahrzeugs nicht geeignet ist.

5. Kraftstoffkreislauf nach einem der vorhergehenden Ansprüche, verbunden mit Mitteln zum Blockieren des Neustartens des Fahrzeugs, wenn der zum Füllen des Tanks verwendete Kraftstoff nicht für den Motor des Fahrzeugs geeignet ist.
